(19) Europäisches Patentamt
European Patent Office
Office européen des brevets

(11) EP 0 498 706 B2

(12) # NOUVEAU FASCICULE DE BREVET EUROPEEN

(45) Date de publication et mention de la décision concernant l'opposition:
**31.05.2000 Bulletin 2000/22**

(45) Mention de la délivrance du brevet:
**08.11.1995 Bulletin 1995/45**

(21) Numéro de dépôt: **92400268.6**

(22) Date de dépôt: **03.02.1992**

(51) Int. Cl.[7]: **C12P 41/00**, C12P 17/02, C12P 7/62

(54) **Procédé de préparation du (-)-(2R,3S)-2,3-époxy-3-(4-méthoxyphényl)propionate de méthyle**

Verfahren zur Herstellung von (-)-(2R,3S)-2,3-Epoxy-3-(4-Methoxyphenyl)propionat-methylester

Process for the preparation of (-)-(2R,3S)-2,3-epoxy-3-(4-methoxyphenyl)methylpropionate

(84) Etats contractants désignés:
**AT BE CH DE DK ES FR GB GR IT LI LU MC NL PT SE**

(30) Priorité: **08.02.1991 FR 9101427**

(43) Date de publication de la demande:
**12.08.1992 Bulletin 1992/33**

(73) Titulaire: **SYNTHELABO**
**92350 Le Plessis Robinson (FR)**

(72) Inventeurs:
- **Zard, Lydia**
  **F-91190 Gif sur Yvette (FR)**
- **Tixidre, Arlette**
  **F-91400 Orsay (FR)**
- **Rossey, Guy**
  **F-78960 Voisins le Bretonneux (FR)**
- **Wick, Alexander**
  **F-78860 Saint Nom la Bretèche (FR)**

(74) Mandataire: **Ludwig, Jacques et al**
**Sanofi-Synthélabo**
**Service Brevets**
**174, avenue de France**
**75013 Paris (FR)**

(56) Documents cités:
**EP-A- 0 343 714**
**EP-A- 0 344 791**
**EP-A- 0 362 556**

- **BIOTECHNOLOGY AND BIOENGINEERING. vol. 26, no. 12, Décembre 1984, NEW YORK US, pp. 1449-1454; Bernard CAMBOU et al.;**

Remarques:
Le dossier contient des informations techniques présentées postérieurement au dépôt de la demande et ne figurant pas dans le présent fascicule.



Printed by Xerox (UK) Business Services
2.16.7 (HRS)/3.6

## Description

La présente invention a pour objet un procédé de préparation du (-)-(2*R*,3*S*)-2,3-époxy-3-(4-méthoxyphényl)propionate de méthyle, de formule (I)

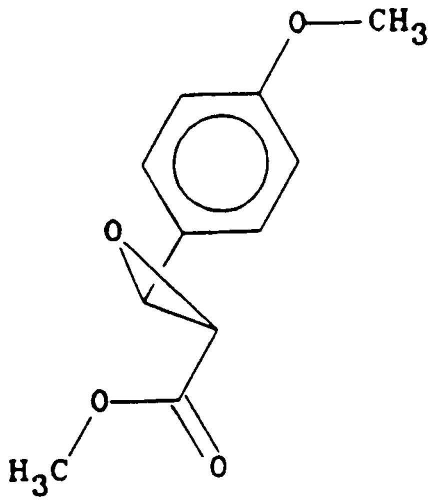

Ce composé est un intermédiaire important dans la préparation de composés tels que la (+)-(2*S*,3*S*)-3-acétyloxy-2,3-dihydro-5-(2-diméthylaminoéthyl)-2-(4-méthoxyphényl)-1,5-5*H*-benzothiazépine-4-one, ou Diltiazem (DCI) et ses dérivés.

Le procédé de l'invention est en fait un procédé de séparation de l'énantiomère lévogyre (2*R*,3*S*) à partir d'un mélange de départ contenant les deux énantiomères de configuration *trans*, par exemple le mélange racémique.

Il consiste en une transestérification de l'énantiomère dextrogyre (2*S*,3*R*), présent dans le mélange de départ, en milieu anhydre et en présence d'une enzyme qui n'affecte pas l'énantiomère lévogyre (2*R*,3*S*).

Le *trans*-(±)-2,3-époxy-3-(4-méthoxyphényl)propionate de méthyle, c'est-à-dire le racémate, est décrit dans *J. Chem. Soc., Perkin Trans. I*, (1984), 1725.

La séparation de l'énantiomère lévogyre du 2,3-époxy-3-(4-méthoxyphényl)propionate de méthyle à partir du racémate en présence d'enzymes a déjà été décrite, par exemple dans les demandes de brevets EP-0343714, EP-0362556 et WO-90/04643.

Les procédés connus mettent en oeuvre des réactions qui sont des destructions hydrolytiques de l'énantiomère dextrogyre, sans que soit affecté l'énantiomère lévogyre ; ils ont pour inconvénient le fait que les produits d'hydrolyse (essentiellement l'acide libre et des produits de décarboxylation), tout comme l'énantiomère lévogyre souhaité, restent en solution dans le milieu réactionnel et, de ce fait, les modes opératoires comprennent nécessairement des étapes de séparation liquide/liquide, des extractions, etc, qui ne jouent pas en faveur du rendement et de la pureté du composé final.

Selon la présente invention, au contraire, l'énantiomère dextrogyre est transformé en un dérivé insoluble, que l'on élimine facilement du mélange réactionnel par une simple filtration, l'énantiomère lévogyre restant en solution dans le filtrat.

Selon l'invention, les agents de transestérification sont des sels alcalins ou alcalinoterreux de l'acide 4-hydroxy-butanoïque, par exemple le sel de sodium, de potassium ou de calcium, car, tout comme le dérivé issu de la transestérification, ils sont insolubles dans le milieu réactionnel.

Le solvant à utiliser doit, bien entendu, être inerte vis-à-vis de l'enzyme ; selon l'invention il s'agit d'un solvant non nucléophile choisi parmi les hydrocarbures, par exemple l'hexane, l'heptane, le cyclohexane, le benzène, le toluène, les xylènes, parmi les solvants chlorés, par exemple le dichlorométhane, le chloroforme, le chlorobenzène, les dichlorobenzènes, parmi les éthers, par exemple l'éther diéthylique, l'éther diisopropylique, le tert.-butylméthyléther, ou parmi les cétones, par exemple la méthylisobutylcétone.

Les enzymes à utiliser selon l'invention sont des lipases ou des estérases, de préférence celles de la classe E.C.3.1.1. Les enzymes préférées sont l'enzyme de ***Candida cylindracea***, l'enzyme de ***Alcaligenes***, l'enzyme de ***Mucor miehei*** et l'enzyme L.P.L., de ***Pseudomonas***, de la société Amano Pharmaceutical Ltd.

Elles peuvent être mises en oeuvre telles quelles, ou fixées sur des supports inertes tels que des membranes.

La réaction de transestérification s'effectue à une température de 10 à 70°C, par exemple à température ambiante ou, de préférence, entre 20 et 60°C, et on surveille son avancement par chromatographie liquide haute performance (CLHP).

Les exemples suivants illustrent en détail des modes opératoires particuliers du procédé selon l'invention.

Exemple 1

On dissout 5 g de trans-(±)-2,3-époxy-3-(4-méthoxyphényl)propionate de méthyle dans 150 ml de toluène et on ajoute 2 g de lipase d'***Alcaligenes*** et 4,6 g de 4-hydroxybutyrate de sodium.
On place le mélange réactionnel sous agitation dans un bain thermostaté à 37°C pendant 40h.
On filtre l'insoluble, en le rinçant avec du toluène.
On évapore le filtrat sous pression réduite, et on lave les cristaux obtenus avec du tert.-butylméthyléther à -10°C.
On isole finalement 2 g de cristaux blancs de (-)-(2*R,*3*S*)-2,3-époxy-3-(4-méthoxyphényl)propionate de méthyle pur. Point de fusion : 86-87°C.
$[\alpha]_D^{22}$ = -201° (c=1 ; MeOH)
ee = 100% (CLHP)
(excès énantiomérique,

$$ee = \frac{[R] - [S]}{[R] + [S]} \times 100\%,$$

[R] et [S] étant les concentrations des deux énantiomères).

Exemple 2

On opère comme décrit dans l'exemple 1, en utilisant diverses enzymes et, après 20h d'agitation, on détermine par CLHP le rapport entre le (-)(2*R*,3*S*)-2,3-époxy-3-(4-méthoxyphényl)propionate de méthyle et son énantiomère dextrogyre (qui n'a pas encore réagi) contenus dans le mélange réactionnel.

Le tableau suivant indique les enzymes utilisées et, pour chacune, le rapport des intensités UV (254 nm).

| Lipase | Rapport |
|---|---|
| *Alcaligenes* | 74 / 23 |
| *Candida cylindracea* | 56 / 43 |
| *Mucor miehei* | 62 / 38 |

## Revendications

1. Procédé de préparation du (-)-(2*R*,3*S*)-2,3-époxy-3-(4-méthoxyphényl)propionate de méthyle, caractérisé en ce que, dans un mélange de départ contenant les énantiomères dextrogyre et lévogyre du *trans*-2,3-époxy-3-(4-méthoxyphényl)propionate de méthyle, on soumet l'énantiomère dextrogyre à une transestérification en milieu anhydre, qui transforme l'énantiomère dextrogyre en un dérivé insoluble dans le milieu réactionnel, à l'aide d'un sel de métal alcalin ou alcalinoterreux de l'acide 4-hydroxybutanoïque, en présence d'une enzyme qui n'affecte pas l'énantiomère lévogyre et qui est choisie parmi *Alcaligenes*, *Candida cylindracea* et *Mucor miehei*.

2. Procédé selon la revendication 1, caractérisé en ce que le mélange de départ est le mélange racémique.

3. Procédé selon la revendication 1, caractérisé en ce qu'on effectue la transestérification à l'aide du 4-hydroxybutanoate de sodium.

4. Procédé selon l'une quelconque des revendications précédentes, caractérisé en ce qu'on effectue la transestérification en présence de l'enzyme de *Candida cylindracea*.

5. Procédé selon l'une quelconque des revendications précédentes, caractérisé en ce qu'on effectue la transestérification en présence de l'enzyme de *Alcaligenes*.

6. Procédé selon l'une quelconque des revendications précédentes, caractérisé en ce qu'on effectue la transestérification en présence de l'enzyme de *Mucor miehei*.

7. Procédé selon l'une quelconque des revendications 4 à 6, caractérisé en ce qu'on ajoute l'enzyme telle quelle au milieu réactionnel.

8. Procédé selon l'une quelconque des revendications 4 à 6, caractérisé en ce qu'on utilise une enzyme fixée sur un support inerte.

9. Procédé selon l'une quelconque des revendications précédentes, caractérisé en ce qu'on utilise un solvant non nucléophile.

10. Procédé selon la revendication 9, caractérisé en ce qu'on utilise un solvant choisi parmi l'hexane, l'heptane, le cyclohexane, le benzène, le toluène, les xylènes, le dichlorométhane, le chloroforme, le chlorobenzène, les dichlorobenzènes, l'éther diéthylique, l'éther diisopropylique, le tert.-butylméthyléther et la méthylisobutylcétone.

11. Procédé selon la revendication 9, caractérisé en ce qu'on utilise comme solvant le toluène.

## Claims

1. Process for the preparation of methyl (-)-(2*R*,3*S*)-2,3-époxy-3-(4-méthoxyphényl)propionate, characterized in that, in a starting mixture containing the dextrorotatory and levorotatory enantiomers of methyl *trans*-(2*R*,3*S*)-2,3-époxy-3-(4-méthoxyphényl)propionate, the dextrorotatory enantiomer is subjected to a transesterification in an anhydrous medium, which converts the dextrorotatory enantiomer into a derivative which is insoluble in the reaction mixture, with the aid of an alkali metal salt or alkaline-earth metal salt of 4-hydroxybutanoic acid, in the presence of an enzyme which does not affect the levorotatory enantiomer and which is chosen from *Alcaligenes*, *Candida cylindracea* and *Mucor miehei*.

2. Process according to claim 1, characterized in that the starting mixture is the racemic mixture.

3. Processs according to claim 1, characterized in that the transesterification is carried out with the aid of sodium 4-hydroxybutanoate.

**4.** Process according to any one of the preceding claims, characterized in that the transesterification is carried out in the presence of the *Alcaligenes* enzyme.

**5.** Process according to any one of the preceding claims, characterized in that the transesterification is carried out in the presence of the *Candida cylindracea* enzyme.

**6.** Process according to any one of the preceding claims, characterized in that the transesterification is carried out in the presence of the *Mucor miehei* enzyme.

**7.** Process according to any one of claims 4 to 6, characterized in that the enzyme is added as such to the reaction mixture.

**8.** Process according to any one of claims 4 to 6, characterized in that an enzyme fixed on an inert support is used.

**9.** Process according to any one of the preceding claims, characterized in that a nonnucleophilic solvent is used.

**10.** Proces according to claim 9, characterized in that a solvent is used which is chosen from hexane, heptane, cyclohexane, benzene, toluene, xylenes, dichloromethane, chloroform, chlorobenzene, dichlorobenzenes, diethyl ether, diisopropyl ether, tert.-butyl methyl ether and methyl isobutyl ketone.

**11.** Process according to claim 9, characterized in that the solvent used is toluene.

**Patentansprüche**

**1.** Verfahren zur Herstellung von (-)-(2*R*,3*S*)-2,3-Epoxy-3-(4-methoxyphényl)-propionsäuremethylester, dadurch gekennzeichnet, daß man in einer Ausgangsmischung, welche die rechtsdrehenden und liksdrehenden Enantiomeren des *trans*-(2*R*,3*S*)-2,3-Epoxy-3-(4-methoxyphényl)-propionsäuremethylesters enthält, das rechtsdrehende Enantiomere in wasserfreiem Medium einer Umesterung unterwirft, welche das rechtsdrehende Enantiomere in ein in dem Reaktionsmedium unlösliches Derivat umwandelt, mit Hilfe eines Alkalimetall- oder Erdalkalimetallsalzes der 4-Hydroxybutansäure, in Gegenwart eines Enzyms, welches das linksdrehende Enantiomere nicht beeinflußt, und welches aus *Alcaligenes*, *Candida cylindracea* und *Mucor miehei* ausgewählt ist.

**2.** Verfahren nach Anspruch 1, dadurch gekennzeichnet, daß die Ausgangsmischung die racemische Mischung ist.

**3.** Verfahren nach anspruch 1, dadurch gekennzeichnet, daß man die Umesterung mit Natrium-4-hydroxybutanoat bewirkt.

**4.** Verfahren nach einem der vorhergehenden Ansprüche, dadurch gekennzeichnet, daß man die Umesterung in Gegenwart des Enzyms von *Candida cylindracea* bewirkt.

**5.** Verfahren nach einem der vorhergehenden Ansprüche, dadurch gekennzeichnet, daß man die Umesterung in Gegenwart des Enzyms von *Alcaligenes* bewirkt.

**6.** Verfahren nach einem der vorhergehenden Ansprüche, dadurch gekennzeichnet, daß man die Umesterung in Gegenwart des Enzyms von *Muco miehei* bewirkt.

**7.** Verfahren nach einem der Ansprüche 4 bis 6, dadurch gekennzeichnet, daß man das Enzym so, wie es ist, zu dem Reaktionsmedium zusetzt.

**8.** Verfahren nach einem der Ansprüche 4 bis 6, dadurch gekennzeichnet, daß man ein an einem inerten Träger gebundenes Enzym verwendet.

**9.** Verfahren nach einem der vorhergehenden Ansprüche, dadurch gekennzeichnet, daß man ein nichtnucleophiles Lösungsmittel verwendet.

**10.** Verfahren nach Anspruch 9, dadurch gekennzeichnet, daß man ein aus Hexan, Heptan, Cyclohexan, Benzol, Toluol, Xylolen, Dichlormethan, Chloroform, Chlorbenzol, Dichlorbenzolen, Diethylether, Diisopropylether, tert.-Butylmethylether und Methylisobutylketon ausgewältes Lösungsmittel verwendet.

**11.** Verfahren nach Anspruch 9, dadurch gekennzeichnet, daß man Toluol als Lösungsmittel verwendet.